(19) Europäisches Patentamt
European Patent Office
Office européen des brevets

(11) Publication number: **0 399 328 B1**

(12) # EUROPEAN PATENT SPECIFICATION

(45) Date of publication of patent specification: **15.11.95**

(51) Int. Cl.[6]: **C12N 15/31**, C12N 15/65, C12N 1/21, C12Q 1/68

(21) Application number: **90109074.6**

(22) Date of filing: **14.05.90**

(54) **Teico-resistance conferring sequence.**

(30) Priority: **23.05.89 EP 89109273**

(43) Date of publication of application:
**28.11.90 Bulletin 90/48**

(45) Publication of the grant of the patent:
**15.11.95 Bulletin 95/46**

(84) Designated Contracting States:
**AT BE CH DE DK ES FR GB GR IT LI LU NL SE**

(56) References cited:
**EP-A- 0 204 549**

**CHEMICAL ABSTRACTS, vol. 104, 1986, page 408, abstract no. 65744f, Columbus, Ohio, US; A.L. BARRY et al.: "Evaluation of teicoplanin and vancomycin disk susceptibility tests", & J. CLIN. MICROBIOL. 1986,23(1), 100-3**

**CHEMICAL ABSTRACTS, vol. 109, 1988, page 408, abstract no. 187086s, Columbus,Ohio, US; R. LECLERCO et al.: "Plasmid-mediated resistance to vancomycin and teicoplanin in Enterococcus faecium", & N. ENGL. J. MED. 1988, 319(3), 157-61**

(73) Proprietor: **GRUPPO LEPETIT S.p.A.**
**Via Roberto Lepetit, 8**
**I-20020 Lainate (MI) (IT)**

(72) Inventor: **Denaro, Maurizio**
**34/21 Sporting Mirasole**
**I-20090 Opera (Milano) (IT)**
Inventor: **Lorenzetti, Rolando**
**137 Via Cavallotti**
**I-20052 Monza (Milano) (IT)**
Inventor: **Moroni, Mariacristina Anna**
**4, Via Trezzo D'Adda**
**I-20144 Milano (IT)**
Inventor: **Sosio, Margherita Amalia**
**3/5 Via Monte Grappa**
**I-20020 Solaro (Milano) (IT)**

(74) Representative: **Macchetta, Francesco et al**
**GRUPPO LEPETIT S.p.A.**
**Patent and Trademark Department**
**Via Roberto Lepetit, 34**
**I-21040 Gerenzano (Varese) (IT)**

**EP 0 399 328 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**FEBS LETTERS, vol. 253, nos. 1,2, August 1989, pages 108-112, Federation of European Biochemical Societies; M.C. MORONI et al.: "Cloning of a DNA region of Actinoplanes teichomyceticus conferring teicoplanin resistance"**

## Description

The present invention relates to a new DNA fragment or a sub-fragment thereof which is capable of conferring resistance to a dalbaheptide antibiotic, upon transfection into a suitable microbial host by a proper vector.

These dalbaheptide antibiotics are a class of antibacterial agents sometimes defined as glycopeptide antibiotics, which are primarily active against gram positive bacteria and includes an increasing number of agents such as vancomycin (US patent 3067099), ristocetin (US patent 4083964), avoparcin (US patents 3338786 and 4322343), teicoplanin (US patents 4239751 and 4542018), actaplanin (US patents 3816618 and 4537715), AAD-216 or ardacin (US patent 4548974), AM 374 (US patent 3803306), A 477 (US patent 3928571), A 40926 (EP-A 177882), A 41030 (US patent 4537770), AAD-609 (EP-A-218416), and A 33512 B (US patent 4029769).

All throughout the description, the term dalbaheptide antibiotic is to be taken as meaning glycopeptide antibiotic.

From a structural point of view these antibiotic substances are characterized by the presence of a core heptapeptidic structure made up of seven amino acids, five of which are constantly aryl- and arylmethylaminoacids, said structure being determinant of a common mechanism of action, i.e. the specific complexation with the D-alanyl-D-alanine terminus of one or more intermediates of the cell wall synthesis. The aryl- and/or arylmethyl portions in most cases bear a few sugar units linked through O-glycosidic bonds. Oftentimes, these sugars are typical of a given dalbaheptide (see F. Parenti and B. Cavalleri: "Proposal to name the vancomycin-ristocetin like glycopeptides as dalbaheptides", The Journal of Antibiotics, Vol. 42, No. 12, pp 1882-1883, December 1989).

The common "mechanism of action" of dalbaheptides involves their binding to the UDP-N-acetylmuramylpentapeptide precursor of the growing bacterial cell-wall, thus impairing the peptidoaglycon polymerization. Like other cell-wall inhibitors, dalbaheptide antibiotics are bactericidal only against actively growing pathogens.

Also included in this class are derivatives of the above mentioned substances which are obtained by selective partial hydrolysis or other chemical modification process. For instance, teicoplanin aglycon and pseudoaglycons are described by A. Malabarba et al. in J. Antibiotics, 37, 988 (1984), 39, 1430 (1986), and EP-A 301247. Carboxy ester derivatives of teicoplanin compounds are described by A. Malabarba et al. in J. Antib. 40, 1572 (1987), while carboxamides are described in EP-A 218099, WO88/06600, or European Pat. Appln. Publication No. 352538 and European Patent Application Ser. No. 89122874.4.

Any of the above defined antibiotics, as well as the derivatives thereof will be referred to in this application as "dalbaheptide antibiotics". This definition includes, of course, the substances that will be added in the future to this expanding class of antimicrobial substances and which share the same features as above.

One object of the present invention is a DNA sequence capable of conferring a resistance to a dalbaheptide antibiotic when used to transform a sensitive host by means of a suitable vector.

As mentioned, the resistance conferred by the DNA sequence of the invention is not specific of a single antibiotic substance but is directed, although with a possibly different degree of efficiency, to any dalbaheptide antibiotic. Even if a mechanism for such a resistance has not be proven so far, a few experiments will be presented below showing a possible way in which the resistance effect conferred by the sequences of the invention manifests itself in a transformed host.

In any case, the present disclosure is not intended as limited to any mechanism of action or theory on it.

For convenience, a DNA sequence of the invention, as defined above, will be referred to as "teico-R conferring sequence".

A particular object of this invention is to provide a "teico-R conferring sequence", the original source of which is a microorganism producing a dalbaheptide antibiotic.

An example of such teico-R conferring sequence is contained in a BamHI fragment of about 2614 bp obtainable as a DNA restriction fragment from the genome of Actinoplanes teichomyceticus ATCC 31121.

This fragment, although derived from the genome of a given producer of a dalbaheptide antibiotic (namely Actinoplanes teichomyceticus ATCC 31121 which produces teicoplanin) is able to confer resistance also to other dalbaheptide antibiotics.

A DNA fragment of the invention is thus a DNA fragment of 2614 bp which can be obtained by BamHI treatment of the genome of A. teichomyceticus ATCC 31121, a DNA sequence containing this fragment, a subfragment thereof or a DNA sequence which, at medium-low stringency hybridizes to a significant extent with the above BamHI fragment, or with a probe derivable from it, and is capable of conferring a resistance to a dalbaheptide antibiotic upon transfection into a sensitive microbial host.

Therefore, the invention encompasses also DNA sequences which hybridize under high stringency conditions with the above mentioned restriction fragment or a sub-fragment thereof and maintain the ability to confer a resistance to a dalbaheptide antibiotic, upon transformation of a sensitive host.

All the above DNA sequences are encompassed by the present definition of teico-R conferring sequence, which therefore includes:

a) a DNA fragment of 2614 bp which can be obtained by BamHI treatment of the genome of A. teichomyceticus ATCC 31121, a subfragment thereof or a DNA sequence containing it, which is capable of conferring a resistance to a dalbaheptide antibiotic upon transformation into a sensitive microbial host;

b) a nucleic acid sequence which is capable of conferring a resistance to a dalbaheptide antibiotic upon transformation into a sensitive microbial host, and hybridizing with a DNA fragment mentioned above under a);

c) portions of DNA inserts hybridizing with the fragments mentioned under a), and capable of conferring resistance to a dalbaheptide antibiotic upon transformation into a sensitive microbial host;

d) DNA sequences which are degenerate as a result of the degenerated genetic code to DNA sequences defined under a) or b) and are capable of conferring a resistance to a dalbaheptide antibiotic upon transformation into a sensitive microbial host.

A teico-R conferring sequence can be used in constructing a vector that confers the teico-R mediated-resistance to dalbaheptide antibiotics upon transformation of a sensitive microbial host.

The following definitions relates to expressions that are commonly understood and used in the art, but which are reported here for convenience:

"A Recombinant DNA Cloning Vector" or "Vector" (for conciseness), is any autonomously replicating or integrating agent, including, but not limited to plasmids, cosmids, phages, etc.

A "Restriction Fragment" is any linear DNA sequence obtained by digesting a DNA with one or more restriction enzymes.

A "Nucleic Acid Sequence" is a polynucleotide sequence of any length, of natural, synthetic or mixed origin either of DNA or RNA. This definition thus includes specifically a cDNA.

A "Sensitive Host Cell" or "Sensitive Microbial Host" is a host cell that cannot grow in the presence of a given antibiotic, unless it is transformed with a resistance conferring sequence.

"Transformant" is a recipient host cell that has undergone transformation, while "Transformation" is the introduction of DNA into a recipient host cell that changes the genotype and results in a change in the recipient cell.

The "Host Cell" may be any of the known prokariotic or eukariotic microorganisms for which a transforming vector is known or can be devised on the basis of the knowledge in the art. Thus, it includes cells of mammalian, avian, amphibian, yeast, and bacterial and vegetal origin. A preferred group of hosts is represented by bacterial strains belonging to the order of the Actinomycetales and to the genera Streptomyces, Actinoplanes, Amycolatopsis, Nocardia, Actinomadura or Kibdelosporangium.

One of the most preferred groups of microbial hosts is represented by the microorganisms which produce any dalbaheptide antibiotic, such as the producers of the dalbaheptide antibiotics reported above.

With the term "Hybridization" the hybridization of nucleic acids is intended, i.e. the process whereby two single-stranded polynucleotides form a double-stranded molecule, with hydrogen bonding between complementary bases in the two strands. Hybridization can take place between complementary strands of both DNA and RNA to produce duplex DNA, duplex RNA or duplex DNA-RNA hybrid molecules. This process makes it possible to identify specific DNA sequences by hybridization with tagged DNA or RNA probes. The conditions of the hybridization are sometimes qualified as "low", "high" or "medium" stringency depending on the concentration of the buffered saline employed, that is generally a sodium chloride/sodium citrate solution (conventionally, SSC).

In particular, "medium-low stringency" refers to concentrations of this solution in the range of 1 x SSC (i.e. a solution 0.15 M sodium chloride and 0.015 M sodium citrate) or more, while "high stringency" refers to concentration in the range of about 0.1-0.5 x SSC, with about 0.2 x SSC (i.e. 0.03 M sodium chloride and 0.003 M sodium citrate) being preferred, at least in some instances.

A teico-R conferring sequence of the invention can be used to confer a selectable resistance to a dalbaheptide antibiotic upon transformation of a sensitive microbial host with a suitable teico-R containing vector. For example, a plasmid vector for Streptomyces lividans (such as that contained in the strain S. lividans, John Innes Collection No. 3131) carrying a selection marker such as the thiostrepton resistance gene can be used to insert a teico-R conferring sequence in a appropriate restriction site to obtain a plasmid with two resistance markers: thiostrepton (thioR) and teicoplanin or another dalbaheptide antibiotic (teicoR). The new plasmid can then be linearized by digestion with a restriction enzyme which possesses at least one recognition site inside the teicoplanin resistance gene (teico-R), thus leaving the origin of replication and the resistance to thiostrepton unaltered. This allows the insertion of a foreign DNA which will destroy the teico-R mediated-resistance to dalbaheptide antibiotics, thus rendering the organisms transformed with the chimeric plasmid selectable for the simultaneous sensitivity to a dalbaheptide antibiotic such as teicoplanin and the resistance to the other marker (e.g. thiostrepton). If the teico-R conferring sequence is a BamHI restriction fragment described above, an internal restriction site that can be conveniently used in this case is the SstI restriction site (c.f. Fig. 1).

According to another embodiment of the invention, a teico-R codifying sequence can be introduced, via an appropriate vector, into an A. teichomyceticus strain or into any other dalbaheptide antibiotic producing strain such as a producer of any of the above listed antibiotics. This transformation of a producer strain may improve the antibiotic production yields by conferring, or more likely, by increasing the degree of resistance of the strain to its antimicrobial product. In many instances in fact, the efficiency of a microbiological process for producing an antibiotic substance is limited by the sensitivity of the producer strain to high concentrations of its own product (c.f. K. Katagiri, J. Antibiotics, 7, 45-52, 1954). This is particularly the case when the resistance mechanism does not involve an enzyme-mediated inactivation of the antibiotic, as it seems to be for the teico-R mediated-resistance.

Antibiotic producing microorganisms have developed a variety of mechanisms to resist to their own products; many of these mechanisms have been studied and fully characterized (see for instance E. Cundliffe, Brit. Med. Bull., 40, 61-67, 1984). However, for the dalbaheptide antibiotics no specific mechanisms of resistance have been described so far in producing strains. Clinical isolates resistant to these antibiotics are emerging (K.L. Rouff,Clin. Microbiol. Newslett., 11, 1-4, 1989) and some transferable resistance has already been described (D.M. Shlaes et al., Antimicrob. Agents Chemother., 33, 198-203, 1989 and R. Leclercq et al., Antimicrob. Agents Chemother., 33, 10-15, 1989), although their mechanism of action has not yet been elucidated. Since the bacterial target of these antibiotics is on the cell-wall, resistance due to an exclusion mechanism was considered "difficult to conceive" by some authors (c.f. R. Leclercq et al., Antimicrob. Agents Chemother., 33, 10-15, 1989 and D. Greenwood, J. Antimicrob. Chemother., 21, suppl. A, 1-13, 1988).

A further use of a teico-R conferring sequence of the invention, such as the BamHI restriction fragment mentioned above or a subfragment thereof, is represented by the use of such a DNA sequence as a probe to fish out, under appropriate hybridization conditions, the corresponding regions from the genoma of producers of dalbaheptide antibiotics other than Actinoplanes teichomyceticus ATCC 31121.

These DNA sequences, once isolated, can be used to transform a sensitive microbial host in order to confer it a resistance to a dalbaheptide antibiotic. In view of their features, these DNA sequences are encompassed by the present definition of teico-R conferring sequences as it is evident to those skilled in the art. For instance, a gene-bank of a producer strain of a dalbaheptide antibiotic, as defined above, can be prepared as known per se in the art and screened using a teico-R conferring sequence of the invention according to any suitable method, such as a Southern blot, a dot blot or other suitable procedures. All the clones which hydridize at medium-low stringency (for example 1xSSC or more) with the probe can be analyzed for their ability to confer resistance against a dalbaheptide antibiotic in a sensistive host. The DNA insert present in the positive clones which confers resistance to a dalbaheptide antibiotic can then be used as described for a teico-R coding sequence, and is in fact encompassed by the definition of teico-R conferring sequence.

Another application of a sequence of the invention is represented by its use as a probe to detect the presence of similar DNA sequences in clinical isolates under medium-low stringency conditions. The positivity to this test might indicate the presence of this kind of resistance in the isolates, thus suggesting the possibility that the tested strains can develop, or have already developed, a teico-R type of resistance against this class of antibiotics.

A teico-R conferring sequence of the invention can be prepared from a DNA extract of A. teichomyceticus ATCC 31121 after partial digestion with a specific restriction enzyme (e.g. BamHI), ligation to a known vector for a given host cell which is naturally sensitive to dalbaheptide antibiotics, selection of the transformed cells which have acquired a resistance to a dalbaheptide antibiotic and isolation of the DNA sequence which is responsible for the acquired resistance. The single process steps are performed according to techniques known per se in the art that need not be discussed in great details here, since they are readily repeatable by the skilled technician on the basis of the information contained in the present disclosure.

Other methods of preparing a teico-R conferring sequence of the invention are apparent to the skilled technician, also in view of the present disclosure and include the use of a sequence such as the above BamHI fragment of A. teichomyceticus or a subfragment thereof, such as the Sst I-Kpn I inner fragment, to fish out DNA sequences that hybridize with it at medium-low stringency (or high stringency) and possess the teico-R conferring capability. Also synthetic probes can be prepared by the conventional methods after sequencing a teico-R-conferring sequence or a portion thereof, if necessary.

Other processes for preparing a teico-R-conferring sequence include copying a RNA sequence into a cDNA which is then used to confer the teico-R mediated-resistance, according to the usual transformation techniques or similarly known techniques.

By evaluating the sensitivity to several antibiotics of hosts transformed with a teico-R-conferring sequence of the invention in comparison with untransformed hosts, it can be seen that the transformants show a cross-resistance to other dalbaheptide antibiotics, while maintaining substantially the same level of sensitivity to the other antibiotics. These experiments may be conducted according to the usual procedures both in liquid and solid growth media.

In addition, by conventional procedures, it is also possible to verify that the hosts transformed with a teico-R-conferring sequence of the invention, when exposed to a dalbaheptide antibiotic, do not modify it chemically in any detectable way, as revealed by HPLC analysis.

By conducting binding assays of dalbaheptide antibiotics on such transformants, data are obtained that indicate a reduced binding of the target antibiotic by the whole cells, under the usual binding conditions.

Brief description of the drawings

A restriction map of a BamHI DNA fragment of 2614 bp of the genomic DNA of Actinoplanes teichomyceticus ATCC 31121 is reported in Fig. 1 of the accompanying drawings.

It reveals an insert containing two Xho II sites at its ends, which were originated by joining BglII and BamHI sticky ends. Only relevant restriction sites are reported in the map drawing.

The line marked in black between the inner Sst I and the Kpn I recognition sites shows the fragment used as probe for the Southern hybridization (c.f. Example 2).

EXAMPLES

The following examples are intended as an illustration of the invention and of the way in which it can be exploited, but cannot be construed as a limitation upon its scope.

Many of the molecular biology methodologies and protocols reported or referred to in the following sections are known per se in the art and are part of the background knowledge of a person of ordinary skill in this art. They are reported also in many reference books and manuals such as: D.A. Hopwood et al., Genetic Manipulation of Streptomyces, a Laboratory Manual, The John Innes Foundation, Norwich, U.K., (1985); T. Maniatis et al., Molecular Cloning, a Laboratory Manual, C.S.H. Laboratory, Cold Spring Harbour, N.Y, (1982) and Current Protocols in Molecular Biology, Greene Publishing Associates and Wiley-Interscience, N.Y, (1987).

To avoid lengthy repetitions of these known techniques that are boring, time-consuming and superfluous to the skilled reader, we will extensively resort to citations of a given reference book or manual, both for known protocols and methodologies. For conciseness, the above mentioned manuals will be referred to respectively as "Hopwood", "Maniatis" and "Current Protocols".

Example 1: Isolation of a teico-R conferring DNA sequence

1.1 The total genomic DNA of Actinoplanes teichomyceticus ATCC 31121 was isolated, partially digested with the restriction endonuclease BamHI and size fractionated by sucrose gradient centrifugation according to the procedures described in Hopwood (see pages 79-80 and 152-153 in particular) and Current Protocols (see in particular Section I, unit 3.1 and specifically 3.1.1 to 3.1.4). The only variations introduced to these protocols relate to the lysozyme solution used in the isolation step that was 5 mg/ml instead of 2 mg/ml and the incubation time of the lyzosyme-treated mycelium that was 2 h instead of 30 min.

1.2 The fractionated 2.5 to 10Kb fragments were then ligated with BglII linearized, phosphatase-treated plasmid pIJ702 (commonly available and contained in S. lividans, John Innes Collection 3131; its restricion map is reported in Hopwood, pages 292-293). This ligation step was carried out essentially according to Hopwood (pages 154-157) with a ratio vector : insert of about 1:5; the linearization and phosphatase-treatment of pIJ702 were carried out following the usual procedures, c.f. Hopwood pages 158-159, 164 and 284-285.

1.3 The obtained ligation mixture was then used to transform S. lividans 66 protoplast (see for instance Hopwood pages 12-14, 110-111 and 236 for further technical or methodological details). After selection with thiostrepton (25 mg/l), about 11000 transformants were obtained which contained recombinant plasmids. S. lividans 66 is deposited in the John Innes Collection under accession number 1326.
1.4 Spore suspensions of these transformants were plated at low density to select for teicoplanin resistance; one clone was selected for the ability to grow in presence of 20 mg/l of the antibiotic. The plasmid present in this clone was named pTR168.

Example 2: Confirmation that the selected plasmid contains the teico-R conferring sequence

2.1 Plasmid pTR168 was extracted (Hopwood, p. 85-91) from the above obtained cells and used to retransform S. lividans 66 protoplast (according to the methodology described in Example 1.3). More than 95% of the thiostrepton resistant transformants were also resistant to teicoplanin, demonstrating that teicoplanin resistance was linked to pTR168.
2.2 The restriction map of the plasmid revealed an insert 2614 bp long (c.f. Fig. 1) containing two Xho II sites at its ends, which were originated by joining BglII and BamHI sticky ends, one SmaI site, restriction sites for SstI, ApaI, SmaI, PvuII and KpnI, and apparently no recognition sites for BamHI, Bcl I, Bgl II, Cla I, Eco RI, Eco RV, Pst I, Sph I, Xho I.
2.3 The inner SstI and KpnI fragment of the teico-R conferring fragments of about 1.5 kb reported above was isolated, radiolabelled and used in a Southern blot hydridization with the genomic DNA of A. teichomyceticus ATCC 31121 digested with various restriction enzymes (KpnI-SstI; Bam HI; Pst I; Pvu II, and Bgl II) according to the known procedures (as reported in Example 1.1). Hybridization is conducted in the conventional way, as described also in the instructions of the membrane manufacturer.
Radiolabelling of the probe was made with $^{32}P$ according to the random-oligonucleotide-primed synthesis (Current Protocols, 3.5.8, 3.5.9), while the genomic DNA, after restriction according to the procedure already reported above, was electrophoretically separated on 1% agarose in TRIS-acetate buffer and transferred onto a nylon membrane. More particularly, the gel containing the separated DNAs band was incubated in 0.4 N NaOH-0.6 M NaCl for 30 min at room temperature to denature the DNA, then was incubated with 1.5 M NaCl-0.5 M TRIS-HCl pH 7.5 for 30 min to neutralize it and transferred onto a nylon membrane (Gene Screen Plus, NEN) prepared in 10 x SSC (1.5 M NaCl-0.15 M sodium citrate). The transfer was allowed to continue overnight in 10XSSC, then the membrane was air-dried, UV treated and the hybridization with the tagged probe was conducted at 65°C according to the known procedures which include pre-hybridizing this membrane by treating it with 10 ml of a solution of 1% sodium dodecyl sulfate, 1 M NaCl, 1% Denhardt's solution and 10% dextrane sulfate in, a sealable plastic bag which was then sealed and incubated with constant agitation for at least 15 min at 65°C. Then 0.5-1 ml of a solution of denaturated salmon sperm DNA (≧ 100 microg/ml) and denaturated tagged probe (final concentration ≦ 10 ng/ml or 1-4 x $10^5$ dpm/ml) were added thereto. The resealed bag was incubated with constant agitation for about 16 h at 65°C. Excess probe was washed away with 0.2 x SSC at 65°C.

The results obtained show that the probe always hybridizes with only one DNA restriction fragment per each digested DNA preparation, apart from the case of the Pvu II digested DNA where two bands were detected. These results are consistent with the hypothesis that the insert in pTR168 is present as a unique sequence in the A. teichomyceticus (ATCC 31121) genome, considering that Pvu II is the only one among the used restriction enzymes which has a site within the probe.

Example 3: Nucleotide sequence of plasmid PTR 168 insert conferring teicoplanin resistance

The entire DNA segment was sequenced by the dideoxy chain termination method, following the procedures described in "Current Protocols" (Chapter 7 from 1.1 to 6.5). For this purpose, the taq DNA Polymerase Sequencing kit (from United States Biochemical Corporation, Cleveland, Ohio, USA) was used (TAQuence ™; Taq DNA Polymerase Sequencing system; step-by-step Protocols for DNA Sequencing with TAQuence, 1st edition, 1989).

In Fig. 2 the arrows indicate the DNA restriction fragments which have been cloned into the phages M13mp18 and M13mp19 (R.M. Dale, B.A. Mc Clure and J.P. Houchins, Plasmid 13, 31-41, 1985) for sequencing analysis and the direction and extent of the DNA region sequenced.

Multiple arrows indicate the DNA fragments sequenced using appropriate oligonucleotides as primers. The analysis of the sequence shows a G + C content of 75.4% which is in the same range of that possessed by other actinomycetes. The sequence is reported under the "Sequence Listing" identified as SEQ ID No. 1.

Example 4: Sensitivity of transformed and untransformed hosts to several antibiotics.

4.1 S.lividans 66 harboring pIJ702 or teico-R conferring pTR168 were tested for their sensitivity to several antibiotics by the antibiogram disc assay. Standard 6 mm antibiogram discs (BBL, Cockeysville, MD, USA) were deposited onto a dense spore suspension of the strains under investigation, and the inhibition zones were measured after 2 days of incubation at 28°C. The results are reported in Table I below.

As expected, the only difference between the two strains was found with teicoplanin which did not inhibit the growth of the cells containing the teico-R conferring pTR168.

TABLE 1

Comparison of the activity of known antibiotics on S.lividans harboring the parental plasmid pIJ702 or the recombinant plasmid pTR168.

| ANTIBIOTIC | µg or (IU)* per disc | inhibition zone (mm) | |
|---|---|---|---|
| | | pIJ702 | pTR168 |
| NEOMYCIN | 30 | 13.5 | 12.5 |
| PAROMOMYCIN | 30 | 15.5 | 14.5 |
| KANAMYCIN | 30 | 22.5 | 21.5 |
| GENTAMYCIN | 10 | 12.5 | 12.5 |
| TOBRAMYCIN | 10 | 14.0 | 13.5 |
| AMIKACIN | 30 | 21.5 | 20.5 |
| ERYTHROMYCIN | 15 | 16.0 | 15.0 |
| NOVOBIOCIN | 30 | 22.5 | 22.5 |
| RIFAMPICIN | 30 | 19.0 | 16.0 |
| BACITRACIN | (10) | 21.0 | 21.0 |
| TEICOPLANIN | 30 | 24.5 | 0 |

* = International Units

4.2 Both strains were also tested on agar plates for their resistance to dalbaheptide antibiotics.

Vancomycin, teicoplanin and antibiotic A 40926 were used in this test.

S. lividans seems to be intrinsically resistant to vancomycin as it grew even on plates containing 100 mg/l of this antibiotic. The cells containing pTR168 grew with 100% plating efficiency in the presence of 100 mg/l of teicoplanin or 50 mg/l of A/40926, while the strain harboring pIJ702 was inhibited by 3.2 mg/l of either antibiotic.

These data suggest that resistance conferred by pTR168 is specific for dalbaheptide antibiotics and is not limited to teicoplanin alone.

Example 5: Teicoplanin binding assays on S. lividans carrying either the parental or the recombinant pTR168 plasmid.

5.1 No chemical modification of teicoplanin was detected by HPLC analysis (c.f. the method described by Riva E. et al. in Chromatographia, 1987, 24, 295-301) of supernatants of resistant (pTR168) or sensitive (pIJ702) S. lividans cultures after up to 18 hours of contact with the antibiotic. However, less teicoplanin was recovered from the culture of sensitive cells, as if these had bound more antibiotic than the resistant ones.

5.2 Streptomyces lividans carrying pIJ702 (hereinbelow identified as S. lividans pIJ702) or S. lividans carrying recombinant plasmid pTR168 (hereinbelow identified as S. lividans-R pTR168) were grown at 30°C into late log phase. Two aliquots of each culture were centrifuged; the supernatants and the resuspended pellets were used to make the following mixtures:

- A) S. lividans (pIJ702) + its own supernatant
- B) S. lividans (pIJ702) + the supernatant from S. lividans-R (pTR168)
- C) S. lividans-R (pTR168) + its own supernatant
- D) S. lividans-R (pTR168) + the supernatant from S. lividans (pIJ702).

To each mixture 10 mg/l of $^{3}$H-labelled teicoplanin (37 kBq) were added followed by incubation at 30 °C for 20 min with agitation. Total input radioactivity was determined at the time of addition of the antibiotic. The incubated cultures were then centrifuged and the radioactivity of the supernatants determined. The pellets were washed with an equal volume of 0.9% NaCl and the radioactivity of the washing solution was determined along with that remaining bound to the cells.

As shown below in Table 2, the sensitive cells bound about half of the teicoplanin which was present in the medium. Regardless of the source of the supernatant, very little antibiotic was removed by washing. The resistant cells, in either supernatant, did not bind significant amounts of antibiotic.

TABLE 2

Teicoplanin binding assay.

Values are expressed as per cent of the total input radioactivity; the sum of each column is not exactly 100% because of the variability in the sampling and counting.

| Mycelium supernatant | A (pIJ702) (pIJ702) | B (pIJ702) (pTR168) | C (pTR168) (pTR168) | D (pTR168) (pIJ702) |
|---|---|---|---|---|
| Broth | 46.5% | 42.7% | 85.0% | 83.1% |
| Washing | 5.5% | 5.6% | 7.2% | 6.7% |
| Cells | 47.9% | 51.6% | 7.6% | 10.0% |

5.3 Another teicoplanin binding experiment was conducted according to a known receptor binding immunoassay (RASA; Corti et al., 1987, Clin. Chem. 39/9, 1615-1618). Cultures of S. lividans harboring pIJ702, pTR168 or uninoculated broth were incubated for 1 hour at 30 °C in the presence of 0.15 to 4.8 mg/l of teicoplanin. After centrifugation, the amount of teicoplanin remaining in the supernatant was determined by RASA. For convenience, a brief description of this method is reported hereinbelow. (See also EP Appln. Publication No. 221282).

The method is based on the selective adsorption of teicoplanin onto microtiter plates coated with albumin-epsilon-aminocaproyl-D-alanyl-D-alanine, and the reaction with anti-teicoplanin antibodies. The complexes are then detected by incubation with anti-antiteicoplanin antibodies, and chromogenic development reaction with o-phenylenediamine. To assay teicoplanin the samples were diluted 1:5 in 20% serum, 20% S/bis broth and 40% RASA buffer (PBS-BTS: Phosphate buffered saline plus 0.5 ml of Tween 20 per liter, 3 g of BSA/liter, and 20% serum). Each well of the microtiter plates, coated in advance with albumin-epsilonaminocaproyl-D-alanyl-D-alanine (Corti et al., see above), was added of 0.1 ml of the diluted sample, and incubated in covered humid box for 2 hours at 30 °C. Then, the plates were washed 8 times with PBS-T (PBS, added with 0.5 ml of Tween 20), and then filled with 0.1 ml of rabbit anti-teicoplanin antiserum diluted 1:250 in PBS-BT (as PBS-BTS above, but without 20% serum). The reaction was conducted at room temperature for 1 hour. After washing, 0.1 ml of peroxidase-conjugated specific goat antibodies to rabbit IgGs (diluted 1:500), were dispensed in each well, and left 1 hour at room temperature. After washing, 0.15 ml of chromogenic peroxidase solution (1 g of o-phenylenediamine and 3.5 mM oxygen peroxidase per liter in 0.1M sodium citrate buffer pH 5) were added. After 30 min at 30 °C, the reaction was stopped by adding 0.05 ml of sulfuric acid. The absorbance at 492 nm was determined in a Titertek Multiskan Photometer (Flow Laboratories). Standards were prepared in the same way, with 10, 40, and 160 ng/ml of teicoplanin. Total binding and a specific binding were determined respectively with 10 mg/ml of teicoplanin in dilution buffer, and with the dilution buffer alone. Again, sensitive cells bound the antibiotic more efficiently than the resistant ones. For all the concentrations tested, more than 85% of the teicoplanin remained, in the supernatants of the cells harboring pTR168 while only 25% remained when cells bearing pIJ702 were used.

The data show that the pTR168 induced resistance is correlated with a reduced binding of teicoplanin to the cells.

## SEQUENCE LISTING

SEQ ID No: 1

Sequence type : nucleotides

Sequence length : 2614 base pairs

strandedness : double

topology : linear

Molecule type : Genomic DNA

Original source organism: Actinoplanes teicomyceticus ATCC 31121

Immediate experimental source: S. lividans plasmid pTR168

Properties: contains a gene conferring teicoplanin resistance

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG CGCGCGGTGG ACCTGCTCGG CGTGACCCGC CAGGAGGCCG  180
TGATGGTCGG CGACGCGGTC ACCGACCTGG CCAGCGCCCG CGCGGCCGGC GTCACCGCGA  240
TCGCCGCCGT CTGGGGCGAG GGCGACCCCG CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

## Claims

### Claims for the following Contracting States : AT, BE, CH, DE, DK, FR, GB, IT, LI, LU, NL, SE

**1.** A teico-R conferring sequence which is a nucleotidic sequence capable of conferring resistance to a glycopeptide antibiotic and is selected from:

a) a DNA fragment of 2614 bp which can be obtained by BamHI treatment of the genome of Actinoplanes teichomyceticus ATCC 31121, a subfragment thereof or a DNA sequence containing it, which is capable of conferring a resistance to a glycopeptide antibiotic upon transformation into a sensitive microbial host;

b) a nucleic acid sequence which is capable of conferring a resistance to a glycopeptide antibiotic upon transformation into a sensitive microbial host, and hybridizing under medium-low stringency conditions with a DNA fragment mentioned above under a),

c) DNA sequences which are degenerated as a result of the degeneration of the genetic code to DNA sequences defined under a) or b) and are capable of conferring a resistance to a glycopeptide antibiotic upon transformation into a sensitive microbial host.

2. A teico-R conferring sequence according to claim 1 which is a DNA fragment of 2614 bp which can be obtained by BamHI treatment of the genome of Actinoplanes teichomyceticus ATCC 31121, a subfragment thereof or a DNA sequence containing it, which is capable of conferring a resistance to a glycopeptide antibiotic upon transformation into a sensitive microbial host.

3. A teico-R conferring sequence according to claim 1 which is a nucleic acid sequence which is capable of conferring a resistance to a glycopeptide antibiotic upon transformation into a sensitive microbial host, and hybridizing under medium-low stringency conditions with a DNA fragment of 2614 bp which can be obtained by BamHI treatment of the genome of Actinoplanes teichomyceticus ATCC 31121, a subfragment thereof or a DNA sequence containing it.

4. A teico-R conferring sequence according to claim 1 which is a portion of a DNA insert hybridizing under medium-low stringency conditions with a DNA fragment of 2614 bp which can be obtained by BamHI treatment of the genome of Actinoplanes teichomyceticus ATCC 31121, a subfragment thereof or a DNA sequence containing it, which is capable of conferring a resistance to a glycopeptide antibiotic upon transformation into a sensitive microbial host.

5. A teico-R conferring sequence as in claim 1 paragraphs b) or c), the original source of which is a microorganism producing a glycopeptide antibiotic other than Actinoplanes teichomyceticus ATCC 31121.

**6.** A teico-R conferring sequence according to claim 1 which is a DNA isolate of the following sequence:

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG CGCGCGGTGG ACCTGCTCGG CGTGACCCGC CAGGAGGCCG  180
TGATGGTCGG CGACGCGGTC ACCGACCTGG CCAGCGCCCG CGCGGCCGGC GTCACCGCGA  240
TCGCCGCCGT CTGGGGCGAG GGCGACCCCG CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

or a fragment thereof capable of codifying the resistance to a glycopeptide antibiotic.

**7.** A recombinant plasmid suited for transformation of a microbial host comprising a teico-R conferring sequence of any of claims 1, 2, 3, 4, 5 and 6.

**8.** A microbial host cell transformed with a recombinant plasmid of claim 7.

**9.** A microbial cell according to claim 8 which is a producer of a glycopeptide antibiotic.

**10.** A process for preparing a teico-R conferring sequence of any of claims 1, 2, 3, 4, 5, and 6 which comprises growing a transformed host cell of claim 8 in a suitable medium, recovering and isolating therefrom the teico-R conferring sequence.

**11.** A process for preparing a teico-R conferring sequence of any of claims 1, 2, 3, 4, 5, and 6 which comprises:

a) extracting the DNA fraction from the genome of a microorganism which is a producer of a glycopeptide antibiotic, partially digesting it with specific restriction enzymes
b) ligating the restriction fragments to a linearized vector for a sensitive host cell
c) selecting the transformed cells by means of the acquired resistance and isolating the teico-R conferring sequence from them.

**12.** A process as in claim 11 wherein the microorganism is A. teichomyceticus ATCC 31121 and the digestion enzyme is BamHI.

**13.** Use of a teico-R conferring sequence of any of claims 1, 2, 3, 4, 5, and 6 to detect, isolate, or purify the corresponding regions, or inserts containing them, from the genome of a microbial cell.

**14.** Use according to claim 13 wherein the microbial cell is a producer of a glycopeptide antibiotic.

**15.** Use of a teico-R conferring sequence of any of claims 1, 2, 3, 4, 5, and 6 to introduce or increase the resistance of a microbial host to a glycopeptide antibiotic.

**16.** Use according to claim 15 wherein the microbial cell is a producer of a glycopeptide antibiotic.

**17.** Use of a teico-R conferring sequence of any of claims 1, 2, 3, 4, 5, and 6 for isolating, identifying or purifying a nucleic sequence capable of conferring a resistance to a glycopeptide antibiotic, or an insert containing it, which comprises hybridizing a probe represented by a tagged teico-R sequence according to any of claims 1, 2, 3, 4, 5, and 6 with a nucleic acid fraction, and optionally isolating the nucleic acid fragments that hybridize with the probe under medium-low stringency conditions.

**Claims for the following Contracting States : GR, ES**

**1.** A process for preparing a teico-R conferring sequence which is a nucleotidic sequence capable of conferring resistance to a glycopeptide antibiotic and is selected from:
a) a DNA fragment of 2614 bp which can be obtained by BamHI treatment of the genome of Actionoplanes teichomyceticus ATCC 31121, a subfragment thereof or a DNA sequence containing it, which is capable of conferring a resistance to a glycopeptide antibiotic upon transformation into a sensitive microbial host;
b) a nucleic acid sequence which is capable of conferring a resistance to a glycopeptide antibiotic upon transformation into a sensitive microbial host, and hybridizing under medium-low stringency conditions with a DNA fragment mentioned above under a),
c) DNA sequences which are degenerated as a result of the degeneration of the genetic code to DNA sequences defined under a) or b) and are capable of conferring a resistance to a glycopeptide antibiotic upon transformation into a sensitive microbial host,
which comprises:
a) extracting the DNA fraction from the genome of a microorganism which is a producer of a glycopeptide antibiotic, partially digesting it with specific restriction enzymes
b) ligating the restriction fragments to a linearized vector for a sensitive host cell
c) selecting the transformed cells by means of the acquired resistance and isolating the teico-R conferring sequence from them.

**2.** A process as in claim 1 wherein the microorganism is A. teichomyceticus ATCC 31121 and the digestion enzyme is BamHI.

**3.** A process as in any of claims 1 and 2 wherein the sequence has the following formula

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG CGCGCGGTGG ACCTGCTCGG CGTGACCCGC CAGGAGGCCG  180
TGATGGTCGG CGACGCGGTC ACCGACCTGG CCAGCGCCCG CGCGGCCGGC GTCACCGCGA  240
TCGCCGCCGT CTGGGGCGAG GGCGACCCCG CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

or a fragment thereof capable of codifying the resistance to glycopeptide antibiotics.

**4.** A process as in any of claims 1 to 3 wherein the restriction fragment is a DNA fragment of 2614 bp and the vector is BglII linearized and phosphatase treated pIJ702

**5.** A process as in any of claims 1 to 4 wherein the sensitive host cell is S. lividans.

**Patentansprüche**
**Patentansprüche für folgende Vertragsstaaten : AT, BE, CH, DE, DK, FR, GB, IT, LI, LU, NL, SE**

**1.** Teico-Resistenz (Teico-R) verleihende Sequenz, die eine Nucleotidsequenz ist, die Resistenz gegen ein Glycopeptid-Antibiotikum verleihen kann und ausgewählt ist aus:

a) einem DNA-Fragment mit 2614 Basenpaaren, das erhalten werden kann durch BamHI-Behandlung des Genoms von Actinoplanes teichomyceticus ATCC 31121, einem Subfragment davon, oder einer dieses enthaltenden DNA-Sequenz, die fähig ist,nach Transformation in einen sensitiven mikrobiellen Wirt Resistenz gegenüber dem Glycopeptid-Antibiotikum zu verleihen;

b) eine Nucleinsäuresequenz, die fähig ist, nach Transformation in einen sensitiven mikrobiellen Wirt Resistenz gegen ein Glycopeptid-Antibiotikum zu verleihen und unter mittleren bis niedrigen Stringenzbedingungen mit einem vorstehend unter a) erwähnten DNA-Fragment zu hybridisieren,
c) DNA-Sequenzen, die als Ergebnis der Degeneration des genetischen Codes zu DNA-Sequenzen gemäß der Definition unter a) oder b) degeneriert sind und fähig sind, nach Transformation in einen sensitiven mikrobiellen Wirt Resistenz gegen ein Glycopeptid-Antibiotikum zu verleihen.

**2.** Teico-R verleihende Sequenz nach Anspruch 1, die ein DNA-Fragment mit 2614 Basenpaaren ist, das erhalten werden kann durch BamHI-Behandlung des Genoms von Actinoplanes teichomyceticus ATCC 31121, einem Subfragment davon oder einer dieses enthaltenden DNA-Sequenz, die nach Transformation in einen sensitiven mikrobiellen Wirt Resistenz gegen ein Glycopeptid-Antibiotikum verleihen kann.

**3.** Teico-R verleihende Sequenz nach Anspruch 1, die eine Nucleinsäuresequenz ist, die nach Transformation in einen sensitiven mikrobiellen Wirt Resistenz gegen ein Glycopeptid-Antibiotikum verleihen kann, und unter mittleren bis niedrigen Stringenzbedingungen mit einem DNA-Fragment mit 2614 Basenpaaren hybridisieren kann, das erhalten werden kann durch BamHI-Behandlung des Genoms von Actinoplanes teichomyceticus ATCC 31121, einem Subfragment davon oder einer dieses enthaltenden DNA-Sequenz.

**4.** Teico-R verleihende Sequenz nach Anspruch 1, die ein Teil einer DNA-Insertion ist, die unter mittleren bis niedrigen Stringenzbedingungen mit einem DNA-Fragment mit 2614 Basenpaaren hybridisiert, das erhalten werden kann durch BamHI-Behandlung des Genoms von Actinoplanes teichomyceticus ATCC 31121, einem Subfragment davon oder einer dieses enthaltenden DNA-Sequenz, die nach Transformation in einem sensitiven mikrobiellen Wirt Resistenz gegen ein Glycopeptid-Antibiotikum verleihen kann.

**5.** Teico-R verleihende Sequenz nach Anspruch 1 b) oder c), deren Ursprung ein ein Glycopeptid-Antibiotikum produzierender Mikroorganismus ist, der nicht Actinoplanes teichomyceticus ATCC 31121 ist.

6. Teico-R verleihende Sequenz nach Anspruch 1, die ein DNA-Isolat mit der folgenden Sequenz ist:

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG CGCGCGGTGG ACCTGCTCGG CGTGACCCGC CAGGAGGCCG  180
TGATGGTCGG CGACGCGGTC ACCGACCTGG CCAGCGCCCG CGCGGCCGGC GTCACCGCGA  240
TCGCCGCCGT CTGGGGCGAG GGCGACCCCG CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

oder ein Fragment davon, das Resistenz gegen ein Glycopeptid-Antibiotikum codieren kann.

7. Rekombinantes Plasmid, das für die Transformation eines mikrobiellen Wirts geeignet ist, umfassend eine Teico-R verleihende Sequenz nach einem der Ansprüche 1, 2, 3, 4, 5 und 6.

8. Mikrobielle Wirtszelle, die mit einem rekombinanten Plasmid nach Anspruch 7 transformiert ist.

9. Mikrobielle Zelle nach Anspruch 8, die ein Glycopeptid-Antibiotikum produziert.

10. Verfahren zur Herstellung einer Teico-R verleihenden Sequenz nach einem der Ansprüche 1, 2, 3, 4, 5 und 6, umfassend das Züchten einer transformierten Wirtszelle nach Anspruch 8 in einem geeigneten Medium, Gewinnung und Isolierung der Teico-R verleihenden Sequenz daraus.

11. Verfahren zur Herstellung einer Teico-R verleihenden Sequenz nach einem der Ansprüche 1, 2, 3, 4, 5 und 6, umfassend:

a) Extraktion der DNA-Fraktion aus dem Genom eines Mikroorganismus, der ein Glycopeptid-Antibiotikum produziert, teilweise Spaltung mit spezifischen Restriktionsenzymen,
b) Ligierung der Restriktionsfragmente zu einem linearisierten Vektor für eine sensitive Wirtszelle,
c) Auswahl der transformierten Zellen mit Hilfe der erworbenen Resistenz und Isolierung der Teico-R verleihenden Sequenz daraus.

**12.** Verfahren nach Anspruch 11, wobei der Mikroorganismus A. teichomyceticus ATCC 31121 ist und das Spaltenzym BamHI ist.

**13.** Verwendung einer Teico-R verleihenden Sequenz nach einem der Ansprüche 1, 2, 3, 4, 5 und 6 zum Nachweis, Isolierung oder Reinigung der entsprechenden Bereiche oder diese enthaltender Insertionen aus dem Genom einer mikrobiellen Zelle.

**14.** Verwendung nach Anspruch 13, wobei die mikrobielle Zelle ein Glycopeptid-Antibiotikum produziert.

**15.** Verwendung einer Teico-R verleihenden Sequenz nach einem der Ansprüche 1, 2, 3, 4, 5 und 6 zur Einführung oder Steigerung der Resistenz eines mikrobiellen Wirts gegen ein Glycopeptid-Antibiotikum.

**16.** Verwendung nach Anspruch 15, wobei die mikrobielle Zelle ein Glycopeptid-Antibiotikum produziert.

**17.** Verwendung einer Teico-R verleihenden Sequenz nach einem der Ansprüche 1, 2, 3, 4, 5 und 6 zur Isolierung, Identifizierung oder Reinigung einer Nucleinsäuresequenz, die Resistenz gegen ein Glycopeptid-Antibiotikum verleihen kann, oder einer diese enthaltenden Insertion, umfassend die Hybridisierung einer Sonde, die eine markierte Teico-R-Sequenz nach einem der Ansprüche 1, 2, 3, 4, 5 und 6 aufweist, mit einer Nucleinsäurefraktion und gegebenenfalls Isolierung der Nucleinsäurefragmente, die mit der Sonde unter mittleren bis niedrigen Stringenzbedingungen hybridisieren.

**Patentansprüche für folgende Vertragsstaaten : GR, ES**

**1.** Verfahren zur Herstellung einer Teico-Resistenz (Teico-R) verleihenden Sequenz, die eine Nucleotidsequenz ist, die Resistenz gegen ein Glycopeptid-Antibiotikum verleihen kann und ausgewählt ist aus:
a) einem DNA-Fragment mit 2614 Basenpaaren, das erhalten werden kann durch BamHI-Behandlung des Genoms von Actinoplanes teichomyceticus ATCC 31121, einem Subfragment davon, oder einer dieses enthaltenden DNA-Sequenz, die fähig ist, nach Transformation in einen sensitiven mikrobiellen Wirt Resistenz gegenüber dem Glycopeptid-Antibiotikum zu verleihen;
b) eine Nucleinsäuresequenz, die fähig ist, nach Transformation in einen sensitiven mikrobiellen Wirt Resistenz gegen ein Glycopeptid-Antibiotikum zu verleihen und unter mittleren bis niedrigen Stringenzbedingungen mit einem vorstehend unter a) erwähnten DNA-Fragment zu hybridisieren,
c) DNA-Sequenzen, die als Ergebnis der Degeneration des genetischen Codes zu DNA-Sequenzen gemäß der Definition unter a) oder b) degeneriert sind und fähig sind, nach Transformation in einen sensitiven mikrobiellen Wirt Resistenz gegen ein Glycopeptid-Antibiotikum zu verleihen,
umfassend:
a) Extraktion der DNA-Fraktion aus dem Genom eines Mikroorganismus, der ein Glycopeptid-Antibiotikum produziert, teilweise Spaltung mit spezifischen Restriktionsenzymen,
b) Ligierung der Restriktionsfragmente zu einem linearisierten Vektor für eine sensitive Wirtszelle,
c) Auswahl der transformierten Zellen mit Hilfe der erworbenen Resistenz und Isolierung der Teico-R verleihenden Sequenz daraus.

**2.** Verfahren nach Anspruch 1, wobei der Mikroorganismus A. teichomyceticus ATCC 31121 ist und das Spaltenzym BamHI ist.

3. Verfahren nach einem der Ansprüche 1 und 2, wobei die Sequenz die folgende Formel hat:

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG TCGCGCGGTGG ACCTGCTCGG CGTGACCCGC CAGGAGGCCG  180
TGATGGTCGG CGACGCGGTC ACCGACCTGG CCAGCGCCCG .CGCGGCCGGC GTCACCGCGA  240
TCGCCGCCGT CTGGGGCGAG GGCGACCCCG. CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

oder ein Fragment davon, das Resistenz gegen Glycopeptid-Antibiotika codieren kann.

4. Verfahren nach einem der Ansprüche 1 bis 3, wobei das Restriktionsfragment ein DNA-Fragment mit 2614 Basenpaaren ist und der Vektor mit BglII linearisierter und mit Phosphatase behandelter pIJ702 ist.

5. Verfahren nach einem der Ansprüche 1 bis 4, wobei die sensitive Wirtszelle S. lividans ist.

**Revendications**

**Revendications pour les Etats contractants suivants : AT, BE, CH, DE, DK, FR, GB, IT, LI, LU, NL, SE**

1. Séquence conférant une téico-R, qui est une séquence nucléotidique capable de conférer une résistance à un antibiotique glycopeptidique et est choisie parmi:

a) un fragment d'ADN de 2 614 pb qui peut être obtenu par traitement par *Bam*HI du génome d'*Actinoplanes teichomyceticus* ATCC 31121, un sous-fragment de celui-ci ou une séquence d'ADN le contenant, qui est capable de conférer une résistance à un antibiotique glycopeptidique, après transformation dans un hôte microbien sensible;
b) une séquence d'acide nuléique qui est capable de conférer une résistance à un antibiotique glycopeptidique après transformation dans un hôte microbien sensible, et de s'hybrider, dans des conditions de stringence moyennefaible, avec un fragment d'ADN mentionné ci-dessus en a);
c) des séquences d'ADN qui sont dégénérées, par suite de la dégénérescence du code génétique, en séquences d'ADN définies en a) ou b) et sont capables de conférer une résistance à un antibiotique glycopeptidique, après transformation dans un hôte microbien sensible.

**2.** Séquence conférant une téico-R selon la revendication 1, qui est un fragment d'ADN de 2 614 pb qui peut être obtenu par traitement par *Bam*HI du génome d'*Actinoplanes teichomyceticus* ATCC 31121, un sous-fragment de celui-ci ou une séquence d'ADN le contenant, qui est capable de conférer une résistance à un antibiotique glycopeptidique après transformation dans un hôte microbien sensible.

**3.** Séquence conférant une téico-R selon la revendication 1, qui est une séquence d'acide nucléique qui est capable de conférer une résistance à un antibiotique glycopeptidique après transformation dans un hôte microbien sensible, et de s'hybrider, dans des conditions de stringence moyenne-faible, avec un fragment d'ADN de 2 614 pb qui peut être obtenu par traitement par *Bam*HI du génome d'*Actinoplanes teichomyceticus* ATCC 31121, un sous-fragment de celui-ci ou une séquence d'ADN le contenant.

**4.** Séquence conférant une téico-R selon la revendication 1, qui est une partie d'un insert d'ADN s'hybridant, dans des conditions de stringence moyenne-faible, avec un fragment d'ADN de 2 614 pb qui peut être obtenu par traitement par *Bam*HI du génome d'*Actinoplanes teichomyceticus* ATCC 31121, un sous-fragment de celui-ci ou une séquence d'ADN le contenant, qui est capable de conférer une résistance à un antibiotique glycopeptidique, après transformation dans un hôte microbien sensible.

**5.** Séquence conférant une téico-R selon la revendication 1, paragraphes b) ou c), dont la source initiale est un micro-organisme producteur d'un antibiotique glycopeptidique, autre qu'*Actinoplanes teichomyceticus* ATCC 31121.

**6.** Séquence conférant une téico-R selon la revendication 1, qui est un isolat d'ADN de séquence suivante:

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG CGCGCGGTGG ACCTGCTCGG CGTGACCCGC CAGGAGGCCG  180
TGATGGTCGG CGACGCGGTC ACCGACCTGG CCAGCGCCCG CGCGGCCGGC GTCACCGCGA  240
TCGCCGCCGT CTGGGGCGAG GGCGACCCCG CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

ou un fragment de celui-ci capable de coder pour la résistance à un antibiotique glycopeptidique.

**7.** Plasmide recombinant approprié à la transformation d'un hôte microbien, comprenant une séquence conférant une téico-R selon l'une quelconque des revendications 1, 2, 3, 4, 5 et 6.

**8.** Cellule hôte microbienne transformée par un plasmide recombinant de la revendication 7.

**9.** Cellule microbienne selon la revendication 8, qui est un producteur d'un antibiotique glycopeptidique.

**10.** Procédé pour la production d'une séquence conférant une téico-R selon l'une quelconque des revendications 1, 2, 3, 4, 5 et 6, comprenant la culture d'une cellule hôte transformée de la revendication 8 dans un milieu approprié, la récupération et l'isolement à partir de ce dernier de la séquence conférant une téico-R.

**11.** Procédé pour la production d'une séquence conférant une téico-R selon l'une quelconque des revendications 1, 2, 3, 4, 5 et 6, comprenant:

a) l'extraction de la fraction d'ADN du génome d'un microorganisme qui est un producteur d'un antibiotique glycopeptidique, et sa digestion partielle par des enzymes de restriction spécifiques,

b) la ligature des fragments de restriction avec un vecteur linéarisé pour une cellule hôte sensible,
c) la sélection des cellules transformées au moyen de la résistance acquise, et l'isolement à partir de celles-ci de la séquence conférant une téico-R.

**12.** Procédé selon la revendication 11, dans lequel le micro-organisme est *A. teichomyceticus* ATCC 31121 et l'enzyme de digestion est *Bam*HI.

**13.** Utilisation d'une séquence conférant une téico-R selon l'une quelconque des revendications 1, 2, 3, 4, 5 et 6, pour détecter, isoler ou purifier les régions correspondantes, ou des inserts les contenant, à partir du génome d'une cellule microbienne.

**14.** Utilisation selon la revendication 13, dans laquelle la cellule microbienne est un producteur d'un antibiotique glycopeptidique.

**15.** Utilisation d'une séquence conférant une téico-R selon l'une quelconque des revendications 1, 2, 3, 4, 5 et 6, pour introduire ou augmenter la résistance d'un hôte microbien à un antibiotique glycopeptidique.

**16.** Utilisation selon la revendication 15, dans laquelle la cellule microbienne est un producteur d'un antibiotique glycopeptidique.

**17.** Utilisation d'une séquence conférant une téico-R selon l'une quelconque des revendications 1, 2, 3, 4, 5 et 6, pour l'isolement, l'identification ou la purification d'une séquence nucléique capable de conférer une résistance à un antibiotique glycopeptidique, ou d'un insert la contenant, qui comprend l'hybridation d'une sonde, représentée par une séquence téico-R marquée selon l'une quelconque des revendications 1, 2, 3, 4, 5 et 6, avec une fraction d'acide nucléique, et éventuellement l'isolement des fragments d'acide nucléique qui s'hybrident avec la sonde, dans des conditions de stringence moyenne-faible.

**Revendications pour les Etats contractants suivants : GR, ES**

**1.** Procédé pour la préparation d'une séquence conférant une téico-R, qui est une séquence nucléotidique capable de conférer une résistance à un antibiotique glycopeptidique et est choisie parmi:
a) un fragment d'ADN de 2 614 pb qui peut être obtenu par traitement par *Bam*HI du génome d'*Actinoplanes teichomyceticus* ATCC 31121, un sous-fragment de celui-ci ou une séquence d'ADN le contenant, qui est capable de conférer une résistance à un antibiotique glycopeptidique, après transformation dans un hôte microbien sensible;
b) une séquence d'acide nuléique qui est capable de conférer une résistance à un antibiotique glycopeptidique après transformation dans un hôte microbien sensible, et de s'hybrider, dans des conditions de stringence moyennefaible, avec un fragment d'ADN mentionné ci-dessus en a);
c) des séquences d'ADN qui sont dégénérées, par suite de la dégénérescence du code génétique, en séquences d'ADN définies en a) ou b) et sont capables de conférer une résistance à un antibiotique glycopeptidique, après transformation dans un hôte microbien sensible,
comprenant:
a) l'extraction de la fraction d'ADN du génome d'un microorganisme qui est un producteur d'un antibiotique glycopeptidique, et sa digestion partielle par des enzymes de restriction spécifiques,
b) la ligature des fragments de restriction avec un vecteur linéarisé pour une cellule hôte sensible,
c) la sélection des cellules transformées au moyen de la résistance acquise, et l'isolement à partir de celles-ci de la séquence conférant une téico-R.

**2.** Procédé selon la revendication 1, dans lequel le micro-organisme est *A. teichomyceticus* ATCC 31121 et l'enzyme de digestion est *Bam*HI.

**3.** Procédé selon la revendication 1 ou 2, dans lequel la séquence à la formule suivante

```
GGATCCGGTT CGCCGTGGCC ACCGGCAAGA GCGGTCCGCG CGCCCGCTCG CTGCTCGCCG   60
GCCTCGCGTA CGCCACCTGT TCGACCACGT CATCGGCTCC GACGAGGTGC GAACGCAAAC  120
CGGCGCCGGA CATCGTGCTG TCGCGGTGG ACCTGCTCGG CGTGACCCGC CAGGAGGCCG  180
TGATGGTCGG CGACGCGGTC ACCGACCTGG CCAGCGCCCG .CGCGGCCGGC GTCACCGCGA  240
TCGCCGCCGT CTGGGGCGAG GGCGACCCCG. CGCAACTGGT GGCCGCGGGC CCGGACGTGG  300
TGGTGGCCAG CCCGGCCGAC GTGCGCGACT GGTGCCGGGC GGCGCGGGCA TGACCACCAC  360
GGCGATGCAG CGGGTACGGG TCGAGCTCGG CGACCGCTCG TACGACGTGC TGATCGGCCC  420
GGGCGTACGG CACCGGCTGG CCGAGCAGGT GGCCCGCACC GGCGCCCGCC GGGCCGTCGT  480
CGTCTCGGCA CGTCCGGCCG GGTGGACGCC CGATCCCGGC GTGCCGCACC TGGTGCTGTC  540
GGCCGAGGAC GGCGAGGCCG GCAAGACCCT GAGCGTGGTG GAGCGCTACT GCCGCCGGTT  600
CGCGCAGTTC GGGCTGTCCC GCACCGACGT GGTGGTCGCG GTGGGCGGCG GATCGACCAC  660
CGACACGGCC GGGCTGGCCG CCGCGCTGTA CCACCGGGGC GTACCGGTGA TCCACCTGCC  720
GACCACGCTG CTGGCCCAGG TCGACGCGAG CGTCGGCGGC AAGACCGGGG CGAACCTGCC  780
GGAGGGCAAG AACCTCGTCG GCGCCTACTG GCAGCCCCGG GCGGTGCTCT GCGACACCGA  840
CTACCTGGCC ACGTTGAGCG CCGCCGAGCT GCGCAGCGGC TACGGCGAGA TCGCCCGCTG  900
CCACTTCATC GGCGCCGGTG ACCTGCGCGG GCGCTCGCAC ACCGAGCAGA TCGCCGCCAG  960
TGTGCGGCGC AAGGCGGAGC TGGTCTCCGC CGACGAGCGG GAGAGCTCCG GCCGGCGGCA 1020
CCTGCTCAAC TACGGCCACA CCCTGGGGCA CGCGCTGGAG GTCGGCACCG GCTTCACGCT 1080
GCGGCACGGC GAGGCGGTGG CGATCGGCAC GGTCTTCGCC GGACGCCTCG CCGGGCACCT 1140
GGGCCGGATC ACGCCGGAGC GGGTCGCCGA GCACGCCGAG GTGGTCGCCC ACTACGGCCT 1200
GTCCGGCCGG CTGCCGTCCG GTGTGGACCC GGCGGCCCTG CTCGCCCTGA TGCGCCGGGA 1260
CAAGAAGGCC ACGACCGGGC TCACGTTCGT CCTCGACGGG CCGCGCGGGG CGAGACGGTC 1320
ACCGACGTAC CCGAGTCCGC GGTCGCCGCC GTGCTGGCCG CGATGCCGCG CGCCGAGTCC 1380
GAGAGCTGAG GTCTGATGCT GCGTACCGAG CTGGTGCAGC CCCCGCACCG GATGCTGATC 1440
CGGCAGGCGG AGCGGTTCGG CCCGAAGACG GCCTTCCGGG ACGGCCGCCG CGCGGTCGGC 1500
TACGCCGATC TGGAGGCGCG GACCCGCCGG CTGGCCGGTC ATCTCGCCCG GCTCGGCGTA 1560
CGCCCGGGCG ACCGGGTCGC GCTGCTGCTG GGCAACCGGG TGGAGACGGT GGAGAGCTAC 1620
TACGCCGCCG CCCGGGCCGG CGCGGTCGCG GTGCCGCTCA ACCCCCGCTC CACCGAGGCG 1680
GAACTGAGCT ACCTGCTGCG CGACTCCGGC GCCACCGCGG TCATCGCCGA GGCGCAGCAC 1740
CTGGACCGGC TCGCCGGCGC CGGCGTACCG GGGCTCACCC TCGTGGTGGT CGGCGACGAC 1800
GGGCCGGCCG GTTCGCACGG CTACGAGGCG ATGGCCACCG GCGAGCCGGG CGTCGCGGCG 1860
TACGACAACC TGGGCCTCGA CGACGTCGCC TGGATGCTCT ACACCTCCGG CACCACCGGC 1920
AGCCCCAAGG GTGTGCTCTC CACCCCGCGC AACTGCCTGT GGTCGGTGTC GGCCAGCTAC 1980
GTGCCGGTGC TCGGGCTGTC CGAGGCGGAC CGGGTGCTGT GGCCGCTGCC GCTGTTCCAC 2040
AGCCTCTCGC ACATCGCCTG CGTGCTGTCC GTCCCGTCGG TGGGCGCCAC CGCCCGGATC 2100
GCCGACGGCC TGTCCGGCGC CGACCTGCTC GACCTCTGGC CGGCGGAGCG GCCCACGGTC 2160
GTCGCCGGCG TGCCCGCCGT CTACCACGAG CTGGTCGCGA GGCGGCCGCC CGGGACTTCA 2220
CCGCGGACGG GCTGCGGGTC GGGCTGGTCG GCGGCGCGAT CACCACGGCG CAGCTGCGCC 2280
AGGCCGTCGA GGACGCTTCG GGGTGCCCCT GGTCGACGTA CGGCAGCACC GAGACTGCGG 2340
CTCGATCGCG ATCAACTGGC CGACCGGCGC CCGGGTCGAG GGCTCTGCGG GCTGCCCGTG 2400
GTGGGCCTGG CCGTCCGGCT CGTCGACGTG CGGACCGGGC GCGACGTGGC CGACGGCGAC 2460
GAGGGCGAGG TCTGGGTCCG CGGCCCGAAC GTGATGGCCG GCTACCACAA CCAGCCGGAG 2520
GCCACCGCCG AGGTGCTGCG CGACGGCTGG TACCACACCG GCGACCTGGC CCGCCGCGAC 2580
CCGGCCGGCT ACCTCACCGT CACCGGGCGG ATCC                            2640
```

ou est un fragment de celle-ci, capable de coder pour la résistance à des antibiotiques de type glycopeptide.

**4.** Procédé selon l'une quelconque des revendications 1 à 3, dans lequel le fragment de restriction est un fragment d'ADN de 2 614 pb et le vecteur est pIJ702 linéarisé par *Bgl*II et traité à la phosphatase.

**5.** Procédé selon l'une quelconque des revendications 1 à 4, dans lequel la cellule hôte sensible est *S. lividans*.

# RESTRICTION MAP OF pTR168

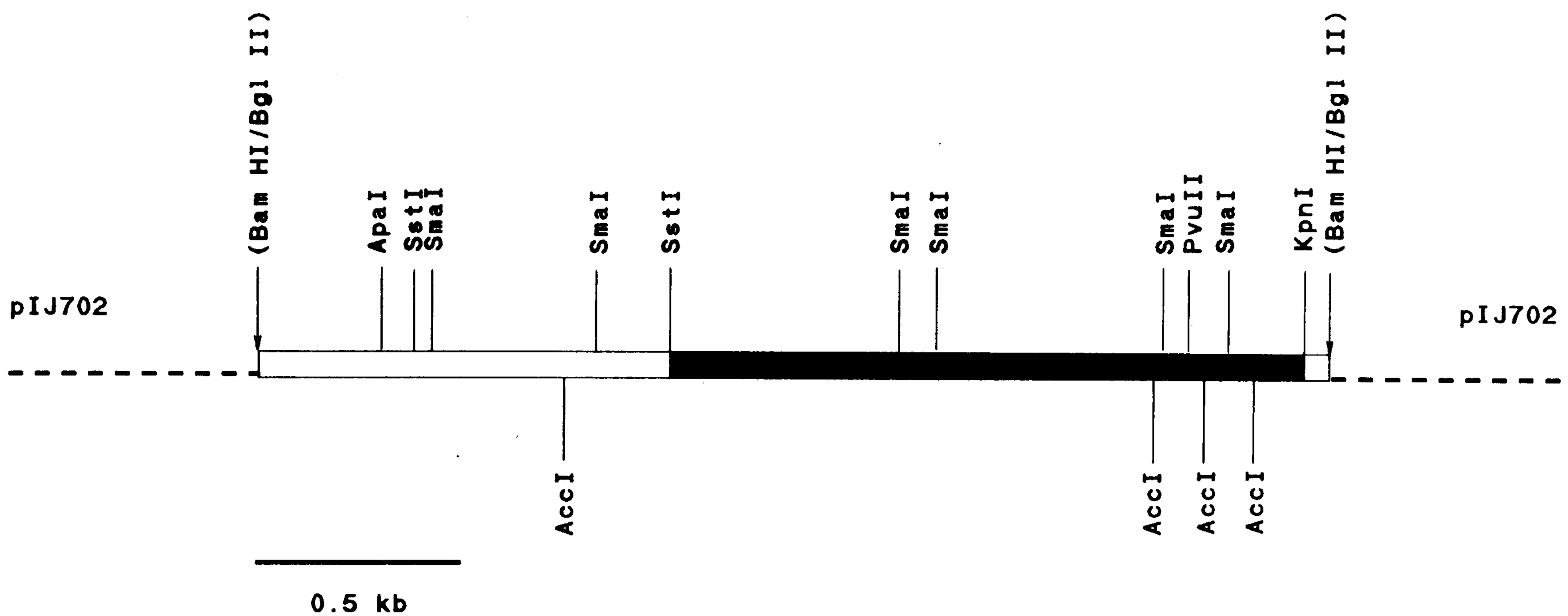


FIG. 1

# SEQUENCING STRATEGY

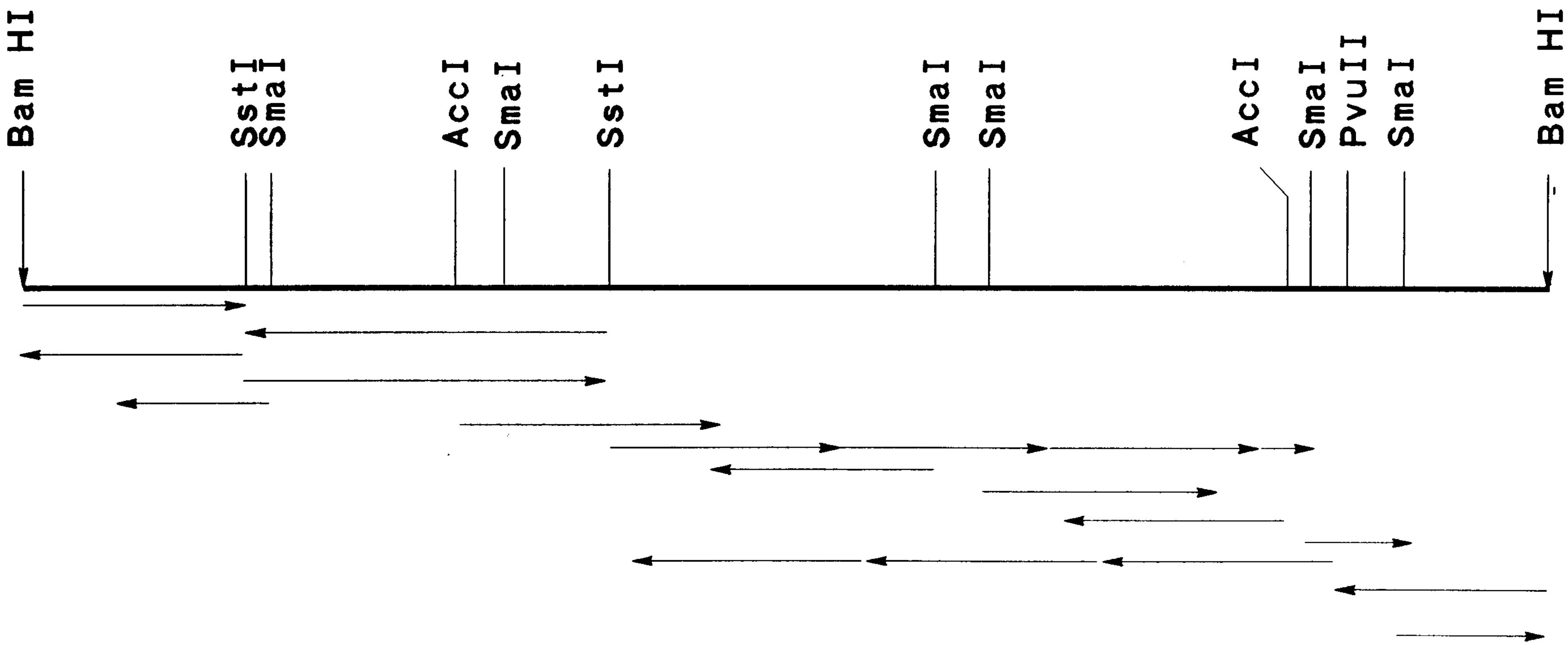


FIG. 2